# FASCICULE DE BREVET EUROPEEN

(11) **EP 0 474 536 B1**
(45) Date de publication et mention de la délivrance du brevet: **28.06.1995**
(21) Numéro de dépôt: 91402290.0
(22) Date de dépôt: 21.08.1991
(51) Int. Cl.: C07C 2/00, C10G 31/00, B01J 29/40, C07C 15/00

(54) **Procédé d'aromatisation des hydrocarbures contenant 2 à 4 atomes de carbone par molécule**
Verfahren zur Aromatisierung von C2-C4-Kohlenwasserstoffen
Process for the aromatisation of hydrocarbons containing 2 to 4 atoms per molecule

(30) Priorité: 03.09.1990 FR 9010946
(43) Date de publication de la demande: 11.03.1992
(73) Titulaire: INSTITUT FRANCAIS DU PETROLE, 92502 Rueil-Malmaison (FR)
(72) Inventeur: Bournonville, Jean-Paul, F-30340 Salindres (FR); Raatz, Francis, F-57500 Saint Avold (FR); Juguin, Bernard, F-92500 Rueil Malmaison (FR)

(56) Documents cités:
- EP-A- 0 018 498
- EP-A- 0 228 267
- WO-A-89/04818
- US-A- 4 766 265

## Description

L'invention concerne l'utilisation d'un catalyseur spécifique dans des réactions d'aromatisation des hydrocarbures comprenant entre 2 et 4 atomes de carbone par molécule.

Le catalyseur comprend une: zéolithe de structure MFI qui contient du silicum et de l'aluminium, et au moins un métal noble de la famille du platine, auxquels sont ajoutés au moins un métal additionnel choisi dans le groupe constitué par l'étain, le germanium, l'indium et le plomb. Une matrice amorphe peut être ajoutée à ce catalyseur en vue notamment de la mise en forme.

La réaction d'aromatisation du propane et du butane en présence d'un catalyseur contenant de la zéolithe ZSM5 (ou MFI) et du gallium a été découverte et brevetée par la société British Petroleum (US-A-4175057 et 4180689). D'autres sociétés ou organismes ont depuis cette date déposé des brevets relatifs à des modifications du solide (US-A-4795844, Brevet Européen EP-B-0252705) et/ou à des changements de charge : coupe C₂-C₁₂ (Brevet Européen EP-B-0252705), éthane et éthylène (Brevet EP-B-0050021 et US-A-4350835). Le mode d'introduction du gallium a fait également l'objet de prise de brevet (Brevets Européens EP-B-0120018 et 0184927).

L'ajout d'un autre métal au système Ga-MFI a été aussi envisagé pour améliorer les sélectivités en aromatiques et pour réduire la quantité de coke sur le catalyseur.

Ainsi l'imprégnation d'un catalyseur Ga/MFI par du rhénium, associé au platine ou au palladium permet d'améliorer de façon significative la sélectivité de production d'aromatiques (US-A-4766265). D'autres brevets revendiquent l'addition de platine et de palladium au catalyseur Ga-MFI (US-A-4407728, Brevets Européens EP-B-0215579, 0216491, 0224162, 0228267 et Brevets Japonais 61268634, 62081329).

L'addition de platine à la zéolithe MFI permet d'améliorer la conversion du propane (T. INUI, F. OKAZUNI, Y. MAKINO, Chem. Express 1 (1), 53-56, 1985). Cependant la sélectivité de production de méthane et d'éthane est sensiblement augmentée. L'adjonction de rhénium au platine accentue encore la production de méthane et d'éthane par hydrogénolyse (S. ENGELS et coll., Catalysis Today, 3, 437-443, 1988). L'ajout de cuivre (P. MERIAUDEAU et coll., Zeolites : Facts, Figures, Future, 1423-29, 1989) et de chrome (E.S. SHPIRO et coll. International Symposium on Zeolites as catalysts, Sorbents and Detergent builders, Würzburg (RFA), p. 73, 1988) diminue la production de méthane, mais la sélectivité en aromatiques reste inférieure aux systèmes Ga/MFI. Par ailleurs, très récemment l'addition de soufre à un catalyseur Pt/MFI permet d'en améliorer sensiblement la sélectivité de production d'aromatiques à partir de paraffines contenant de 6 à 12 atomes de carbones (US-A-4835336).

Par ailleurs dans la demande EP-A-0 458 674 (qui appartient à l'état de la technique selon l'Article 54(3)CBE) la demanderesse revendique un catalyseur basé sur l'association entre une zéolithe MFI et un support (ou une matrice) généralement amorphe sur lequel est déposé un métal noble de la famille du platine et au moins un métal additionnel choisi dans le groupe constitué par l'étain, le germanium, l'indium et le plomb, ledit support contenant également au moins un métal alcalin ou au moins un métal alcalino-terreux choisi dans le groupe constitué par le lithium, le sodium, le potassium, le rubidium, le césium, le baryum, le calcium, le beryllium, le magnésium et le strontium. La demanderesse revendique une amélioration des performances par rapport à ce qui est connu dans l'art antérieur en ce qui concerne l'aromatisation des hydrocarbures légers.

On a découvert que l'introduction du métal noble de la famille du platine (métal hydrogénant) et du métal additionnel directement sur la zéolithe MFI, par des techniques telles que l'imprégnation, l'échange ou toute technique connue de l'homme de l'art, conduit à une amélioration des performances catalytiques notamment par rapport à celles présentées dans le demande de brevet EP-A-0 458 674

La zéolithe de structure MFI du catalyseur (qui de préférence est acide) de la présente invention peut être préparée par toutes les techniques connues dans l'art antérieur. La synthèse de ladite zéolithe MFI peut être réalisée en milieu classique OH⁻, en présence ou non de structurants organiques et/ou d'alcool. La synthèse de la zéolithe MFI en milieu OH⁻ selon les techniques connues dans l'art antérieur est largement décrite dans le document suivant : Synthesis of High Silica Zeolites, P. Jacobs and J. Martens, Studies in Surface Science and Catalysis, Volume 33, Elsevier editor, 1987. La zéolithe MFI peut également être synthétisée dans des milieux moins classiques comme par exemple le milieu fluorure (Brevet Européen EP-A-172068 de C.F.R.).

Après synthèse, la zéolithe MFI est transformée en une forme hydrogène par élimination totale ou partielle des composés organiques et/ou des cations alcalins ou alcalino-terreux qu'elle contient éventuellement après la synthèse. Toutes les techniques connues dans l'art antérieur peuvent être utilisées pour passer à la forme hydrogène, comme par exemple les calcinations sous atmosphère oxydante ou non, les échanges ioniques suivis ou non de calcination, les traitements chimiques divers etc.

Toutes les zéolithes MFI synthétisées dans le système Si-Al ayant le rapport atomique Si/Al compris entre 25 et 200 conviennent pour la présente invention.

Le métal hydrogénant est ensuite déposé sur la zéolithe MFI. N'importe quel métal du groupe VIII de la classification périodique des éléments peut convenir, mais le platine est le métal préféré.

Le platine peut être introduit de différentes façons, par exemple :
- sous forme de complexe tétrammine par échange cationique ;
- sous forme d'acide hexachloroplatinique par imprégnation.

Le platine (ou éventuellement un autre métal noble du groupe du platine) peut être ainsi incorporé dans la zéolithe par imprégnation de cette zéolithe à l'aide d'une solution adéquate aqueuse ou non renfermant un sel ou un composé du métal noble. Le platine est généralement introduit dans la zéolithe sous forme d'acide chloroplatinique, mais peuvent également être utilisés des composés tels que le chloroplatinate d'ammonium, le dichlorure de platine dicarbonyle, l'acide hexahydroxyplatinique, le chlorure de palladium, le nitrate de palladium.

Parmi les composés du ou des métaux de la famille du platine que l'on emploie dans la présente invention, on peut citer également à titre d'exemple les complexes ammoniés.

On citera en particulier dans le cas du platine : les sels de platine IV hexammine de formule (Pt(NH₃)₆)X₄ où X est un atome d'halogène choisi dans le groupe formé par le fluor, le chlore, le brome et l'iode et de préférence X est un atome de chlore ; les sels de platine IV halogénopentammines de formule (PtX(NH₃)₅)X₃ ; les sels de platine IV tétrahalogénodiammines de formule Pt X₄(NH₃)₂ où X a la signification donnée ci-dessus ; les complexes du platine avec les halogènes-polycétones et les composés halogénés polycétoniques de formule H(Pt(aca)₂X) dans lesquels X a la signification donnée ci-dessus et aca représente le reste de formule C₅H₇O₂ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables on peut citer les hydrocarbures paraffiniques, naphténiques ou aromatiques et les composés organiques halogénés ayant par exemple de 1 à 12 atomes de carbone dans leur molécule. On citera en particulier le n-heptane, le méthylcyclohexane, le toluène et le chloroforme. On peut aussi utiliser des mélanges de ces solvants.

L'élément (ou métal additionnel M) choisi dans le groupe constitué de l'étain, du germanium, du plomb et de l'indium peut être introduit par l'intermédiaire de composés tels que par exemple les chlorures, les bromures et le nitrate d'étain, les halogénures, nitrate, acétate et carbonate de plomb, le chlorure et l'oxalate de germanium, le nitrate et le chlorure d'indium.

Le métal additonnel M peut être introduit avant ou après l'introduction du métal noble. S'il est introduit avant le métal noble, le composé utilisé sera choisi dans le groupe constitué par les halogénures, nitrates, acétates, carbonates, oxalates du métal additionnel. L'introduction sera avantageusement effectuée en solution aqueuse. Dans ce cas, avant de procéder à l'introduction du métal noble, on procède à une calcination sous air à une température comprise entre 400 et 1000 degrés centigrades.

Le métal additionnel M peut être introduit après l'introduction du métal noble sous la forme d'au moins un composé organique choisi dans le groupe formé par les complexes, en particulier les complexes polycétoniques, des métaux M et les hydrocarbylmétaux tels que les alkyles, les cycloalkyles, les aryles, les alkylaryles et les arylalkyles métaux.

L'introduction du métal M est avantageusement effectuée à l'aide d'une solution dans un solvant organique du composé organométallique dudit métal M. On peut également employer des composés organohalogénés des métaux M. Comme composés de métaux M on citera en particulier le tétrabutyl-étain, le tétraméthyl-étain, le tétrapropyl-germanium, le tétraéthyl-plomb, l'acétylacétonate d'indium, le triphénylindium.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut utiliser des mélanges des solvants définis ci-dessus.

Cette méthode d'introduction du métal M a déjà été décrite dans le brevet US-A-4548918. Mais la combinaison de la méthode d'introduction du métal de la famille du platine et de la méthode d'introduction du métal M engendre une synergie particulière.

La zéolithe MFI du catalyseur employé dans l'invention renferme en poids (a) environ 0,01 à 2 % et plus particulièrement environ 0,1 à 0,5 % d'au moins un métal noble de la famille du platine, (b) environ 0,005 à 0,60 %, et de préférence 0,01 à 0,50 % d'étain et/ou 0,005 à 0,70 % de préférence environ 0,01 à 0,60 % et plus particulièrement 0,02 à 0,50 % d'au moins un métal choisi dans le groupe constitué par le germanium, le plomb et l'indium.

Lorsqu'il y a au moins deux métaux de la famille étain, germanium, plomb et indium, la teneur globale en métaux de cette famille est d'environ 0,02 à 1,20 % et de préférence 0,02 à 1,0 % et plus particulièrement de 0,02 à 0,8 %.

On peut utiliser soit une solution commune des métaux que l'on désire déposer sur la zéolithe, soit des solutions distinctes pour le métal de la famille du platine et pour le ou les métaux additionnels. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

A l'issue de la préparation du catalyseur, celui-ci est généralement calciné entre 450 et 1000°C mais le catalyseur, à l'issue de la calcination peut subir avantageusement un traitement d'activation sous hydrogène à haute température, par exemple 300-500°C, afin d'obtenir une phase métallique plus active. La procédure de ce traitement sous hydrogène consiste par exemple en une montée lente de la température sous courant d'hydrogène jusqu'à la température maximale de réduction, comprise par exemple entre 300 et 500°C et de préférence entre 350 et 450°C, suivie d'un maintien pendant 1 à 6 heures à cette température.

Ce type de préparation du catalyseur conduit à un solide dans lequel les métaux sont répartis de façon homogène dans tout le volume du grain de catalyseur, et sont dans un état métallique après le traitement de réduction sous balayage d'hydrogène entre 300 et 500°C et maintien pendant 1 à 6 heures sous hydrogène à la température finale choisie.

Une méthode avantageuse de préparation des catalyseurs peut être utilisée selon les étapes suivantes :
(a) On imprègne la zéolithe MFI avec une solution aqueuse d'un composé d'un métal choisi dans le groupe constitué par l'étain, le germanium, l'indium et le plomb ;
(b) On sèche le produit obtenu à l'étape (a) ;
(c) On calcine le produit obtenu à l'étape (b) ;
(d) On imprègne le produit obtenu à l'étape (c) par une solution d'acétylacétonate de platine dans le toluène ;
(e) On sèche le produit obtenu à l'étape (d) ;
(f) On calcine le produit obtenu à l'étape (e) ;
(g) On réduit sous courant d'hydrogène le produit obtenu à l'étape (f).

Une autre méthode avantageuse de préparation des catalyseurs peut être réalisée selon les étapes suivantes :
(a) On imprègne la zéolithe MFI avec une solution aqueuse d'un composé d'un métal choisi dans le groupe constitué par l'étain, l'indium, le germanium et le plomb ;
(b) On sèche le produit obtenu à l'étape (a) ;
(c) On calcine le produit obtenu à l'étape (b) ;
(d) On imprègne avec une solution ammoniacale de chlorure de platine tétrammine le produit obtenu à l'étape (c) ;
(e) On sèche le produit obtenu à l'étape (d) ;
(f) On calcine le produit obtenu à l'étape (e) ;
(g) On réduit le produit obtenu à l'étape (f) sous un courant d'hydrogène.

Une autre méthode avantageuse de préparation des catalyseurs peut être réalisée selon les étapes suivantes :
(a) On imprègne la zéolithe MFI avec une solution ammoniacale de chlorure de platine tétrammine ;
(b) On sèche le produit obtenu à l'étape (a) ;
(c) On calcine le produit obtenu à l'étape (b) ;
(d) On réduit sous courant d'hydrogène le produit obtenu à l'étape (c) ;
(e) On met en contact le produit obtenu à l'étape (d) avec un solvant hydrocarboné et avec ledit composé organique dudit métal M, par exemple en immergeant la masse dans un solvant hydrocarboné renfermant déjà le composé organique ou en immergeant la masse dans un solvant hydrocarboné et en injectant ensuite dans le mélange obtenu une solution du composé organique dudit métal M dans un solvant hydrocarboné et par exemple celui dans lequel ladite masse a été immergée ;
(f) On réduit sous courant d'hydrogène le produit obtenu à l'étape (e).

Le catalyseur peut également contenir un support ou une matrice amorphe, choisi, par exemple, parmi les oxydes de magnésium, d'aluminium, de titane, de zirconium, de thorium, de silicium, de bore, pris seuls ou en mélange entre eux. On peut aussi utiliser du charbon.

Le support préféré est l'alumine ; la surface spécifique de l'alumine peut avantageusement être comprise entre 50 et 600 m²/g, de préférence entre 150 et 400 m²/g.

La zéolithe contenant les divers métaux précédemment décrits peut être mise en forme avec le support par toute technique connue de tout homme du métier : pastillage, extrusion, dragéification, coagulation en goutte, séchage par atomisation par exemple.

Le catalyseur contient alors 1 à 99 % en poids de zéolithe contenant les différents métaux, le complément à 100 % étant constitué par le support ou la matrice amorphe.

Le catalyseur selon l'invention peut notamment être utilisé dans les procédés d'aromatisation d'hydrocarbures renfermant 2 à 4 atomes de carbone par molécule dans les conditions opératoires habituelles desdits procédés.

Les exemples suivants illustrent l'invention sans en limiter la portée.

### EXEMPLE 1

On se propose de transformer du propane en présence d'un catalyseur à base d'une zéolithe MFI de rapport atomique Si/Al égal à 30 contenant du platine et un métal choisi dans le groupe constitué-par l'étain, le germanium, l'indium et le plomb.

### . Préparation de la zéolithe MFI :

La zéolithe MFI est synthétisée en présence de structurant organique en utilisant une des recettes connues dans l'art antérieur (US-A-3.702.886). Cette zéolithe est transformée en une forme H par les traitements suivants :
- calcination sous un mélange air-azote (10 % d'oxygène dans le mélange) à 550°C pendant 4 heures,
- trois échanges dans NH₄NO₃ 5N à 100°C,
- calcination sous air à 530°C pendant 5 heures sous un débit de 5 l/h/g.

Le rapport atomique Si/Al de la zéolithe HMFI est égal à 30, son volume poreux mesuré par adsorption d'azote à 77 K est supérieur à 0,160 cm³/g.

Les métaux sont déposés sur la zéolithe MFI selon les procédures suivantes :

### . Catalyseur A

(a) on imprègne la zéolithe MFI par une solution aqueuse de chlorure d'étain de façon à ce que la concentration finale du catalyseur en étain soit égale à 0,25 % poids.
(b) On sèche le produit obtenu à l'étape (a) pendant 1 heure à 100-120°C.
(c) On calcine le produit obtenu à l'étape (b) pendant 2 heures à 530°C.
(d) On imprègne le produit obtenu à l'étape (c) par une solution aqueuse d'acide hexachloroplatinique de façon à obtenir 0,3 % de platine sur le catalyseur final.
(e) On sèche une heure à 100-120°C le produit obtenu.
(f) On calcine 2 heures à 530°C.

Catalyseur A : 0,3 % Pt et 0,25 % Sn/MFI.

### . Catalyseur B

On imprègne la zéolithe MFI selon la même procédure que pour le catalyseur (A) si ce n'est que l'on remplace le chlorure d'étain par l'acétate d'étain. La suite de la procédure demeure inchangée.
Catalyseur B : 0,3 % Pt et 0,25 % Sn/MFI.

### . Catalyseur C

On imprègne la zéolithe MFI selon la même procédure que pour le catalyseur B si ce n'est que le platine est imprégné au moyen d'une solution ammoniacale de chlorure de platine tétrammine. Le reste de la procédure demeure inchangé.
Catalyseur C : Pt : 0,3 % et Sn : 0,25 %/MFI.

### . Catalyseur D (catalyseur comparatif)

On imprègne la zéolithe MFI par une solution ammoniacale de chlorure de platine tétrammine, de façon à obtenir une concentration en platine égale à 0,3 % poids sur le catalyseur final.
Catalyseur D : Pt : 0,3 %/MFI.

### Exemple 2

On a soumis les catalyseurs préparés ci-dessus à un test d'aromatisation du propane dans les conditions suivantes, ainsi que la zéolithe MFI seule :

| | |
|---|---|
| - Température | 450°C |
| - Pression | 0,1 Mégapascal |
| - pph | 0,5 h⁻¹ |
| - Charge | C₃H₈ |

Les résultats des tests sont donnés dans le tableau 1.

**Tableau 1**

| Catalyseur | Conversion (% mole) | Sélectivité (% mole) | | | |
|---|---|---|---|---|---|
| | | CH₄ | C₂H₆ + C₂H₄ | C₃H₆ | Aromatiques |
| Zéolithe MFI | 14 | 45 | 25 | 30 | 0 |
| Catalyseur D | 40 | 40 | 30 | 10 | 20 |
| Catalyseur A | 31 | 5 | 20 | 35 | 40 |
| Catalyseur B | 32 | 5 | 15 | 30 | 50 |
| Catalyseur C | 34 | 5 | 15 | 25 | 55 |

### Exemple 3

Pour préparer des catalyseurs E, F et G respectivement, on suit une procédure d'imprégnation strictement identique à celle retenue pour la préparation du catalyseur C si ce n'est que l'on remplace l'acétate d'étain par l'oxalate de germanium (catalyseur E), le nitrate de plomb (catalyseur F) et le nitrate d'indium (catalyseur G), le reste de la procédure étant inchangé.
- Catalyseur E : Pt = 0,3 % et Ge : 0,20 %/MFI
- Catalyseur F : Pt = 0,3 % et Pb : 0,35 %/MFI
- Catalyseur G : Pt = 0,3 % et In : 0,25 %/MFI
Dans le tableau 2 sont repris l'ensemble des résultats des tests d'aromatisation de propane réalisés avec ces catalyseurs dans les mêmes conditions que celles définies dans l'exemple 2.

**Tableau 2**

| Catalyseur | Conversion (% mole) | Sélectivité (% mole) | | | |
|---|---|---|---|---|---|
| | | CH₄ | C₂H₆ + C₂H₄ | C₃H₆ | Aromatiques |
| Catalyseur D | 40 | 40 | 30 | 10 | 20 |
| Catalyseur C | 34 | 5 | 15 | 25 | 55 |
| Catalyseur E | 32 | 5 | 17 | 26 | 52 |
| Catalyseur F | 31 | 5 | 18 | 24 | 53 |
| Catalyseur G | 33 | 5 | 16 | 28 | 51 |

### Exemple 4

Le catalyseur D préparé ci-dessus est réduit sous courant d'hydrogène pendant deux heures à 450°C ; on immerge 100 grammes de ce catalyseur dans 300 cm³ de n-heptane. Ensuite on injecte, dans le n-heptane contenant le catalyseur, 2 grammes d'une solution de tétra n-butyl étain dans le n-heptane (à 10 % en étain). Le contact entre le catalyseur au platine et la solution de tétra n-butyl étain est maintenu pendant 6 heures à la température de reflux de l'heptane. La solution d'imprégnation est ensuite évacuée et l'on procède à 3 lavages par du n-heptane pur à la température de reflux du n-heptane. Le catalyseur est alors séché. Il peut ensuite subir soit une calcination sous air pendant 2 heures à 500°C suivie d'une réduction sous courant d'hydrogène à 450°C pendant deux heures avant d'être chargé dans le réacteur, soit subir une réduction directe sous courant d'hydrogène à 450°C pendant 2 heures avant d'être chargé dans le réacteur.

On obtient ainsi le catalyseur H :
Catalyseur H : Pt : 0,3 % et Sn : 0,2 %/MFI
Les résultats du test d'aromatisation du propane, dans des conditions identiques à celles de l'exemple 2 sont rassemblés dans le tableau 3.

**Tableau 3**

| Catalyseur | Conversion (% mole) | Sélectivité (% mole) | | | |
|---|---|---|---|---|---|
| | | CH₄ | C₂H₆ + C₂H₄ | C₃H₆ | Aromatiques |
| Catalyseur D | 40 | 40 | 30 | 10 | 20 |
| Catalyseur C | 34 | 5 | 15 | 25 | 55 |
| Catalyseur H | 36 | 5 | 14 | 24 | 57 |

Les catalyseurs selon toute invention (A, B, C, E, F, G et H) permettent d'obtenir une bonne sélectivité en produits aromatiques avec une sélectivité limitée en méthane.

## Revendications

1. Procédé d'aromatisation d'hydrocarbures renfermant 2 à 4 atomes de carbone par molécule en présence d'au moins un catalyseur renfermant une zéolithe de structure MFI acide et de rapport atomique Si/Al compris entre 25 et 200, contenant au moins un métal noble de la famille du platine et au moins un métal additionnel choisi dans le groupe constitué par l'étain, le germanium, le plomb et l'indium.

2. Procédé selon la revendication 1 dans lequel ladite zéolithe contient au moins un métal noble de la famille du platine en une quantité comprise entre 0,01 % et 2 % poids et au moins un métal additionnel choisi dans le groupe constitué par l'étain, le germanium, le plomb et l'indium en une quantité comprise entre 0,005 et 0,60 % poids pour l'étain et en une quantité comprise entre 0,005 et 0,70 % poids pour le germanium, le plomb ou l'indium.

3. Procédé selon l'une des revendications 1 et 2 dans lequel ladite zéolithe contient au moins un métal noble de la famille du platine en une quantité comprise entre 0,1 et 0,5 % poids et au moins un métal additionnel choisi dans le groupe constitué par l'étain, le germanium, le plomb et l'indium en une quantité comprise entre 0,01 et 0,50 % poids pour l'étain et en une quantité comprise entre 0,01 et 0,60 % poids pour le germanium, le plomb ou l'indium.

4. Procédé selon l'une des revendications 1 à 3 dans lequel ladite zéolithe contient à titre de métaux additionnels de l'étain et au moins un métal choisi dans le groupe constitué par le germanium, le plomb et l'indium, la teneur totale en métaux additionnels de ladite zéolithe étant comprise entre 0,02 et 1,20 % poids.

5. Procédé selon l'une des revendications 1 à 4 renfermant en outre une matrice.

6. Procédé selon la revendication 5 renfermant en poids 1 à 99 % de ladite zéolithe et 99 à 1 % de ladite matrice.

7. Procédé selon l'une des revendications 5 et 6 dans laquelle ladite matrice est l'alumine.

## Claims

1. Process for the aromatization of hydrocarbons containing 2 to 4 carbon atoms per molecule in the presence of at least one catalyst containing an acid MFI structure zeolite which atomic ratio Si/Al is between 25 and 200, containing at least one noble metal from the platinum family and at least one additional metal chosen from within the group constituted by tin, germanium, lead and indium.

2. Process according to claim 1, wherein said zeolite contains at least one noble metal from the platinum family in a quantity of approximately 0.01 to 2% by weight and at least one additional metal chosen from within the group constituted by tin, germanium, lead and indium in a quantity of approximately 0.005 to 0.60% by weight for the tin and a quantity of approximately 0.005 to 0.70% by weight for the germanium, lead or indium.

3. Process according to one of the claims 1 and 2, wherein said zeolite contains at least one noble metal from the platinum family in a quantity of approximately 0.1 to 0.5% by weight and at least one additional metal chosen from within the group constituted by tin, germanium, lead and indium in a quantity of approximately 0.01 to 0.50% by weight for the tin and in a quantity of approximately 0.01 to 0.60% by weight for the germanium, lead or indium.

4. Process according to any one of the claims 1 to 3, wherein said zeolite contains as additional metals tin and at least one metal chosen from within the group constituted by germanium, lead and indium, the total additional metal content of said zeolite being about 0.02 to 1.20% by weight.

5. Process according to any one of the claims 1 to 4 also containing a matrix.

6. Process according to claim 5 containing by weight 1 to 99% of said zeolite and 99 to 1% of said matrix.

7. Process according to either of the claims 5 and 6, wherein said matrix is alumina.

## Patentansprüche

1. Verfahren zur Aromatisierung von Kohlenwasserstoffen, welche 2 bis 4 Kohlenstoffatome im Molekül umfassen, in Gegenwart wenigstens eines Katalysators, umfassend einen sauren Zeolith mit MFI-Struktur und einem Atomverhältnis Si/Al, das zwischen 25 und 200 liegt, der wenigstens ein Edelmetall der Platinfamilie und wenigstens ein zusätzliches Metall enthält, das aus der durch Zinn, Germanium, Blei und Indium bestehenden Gruppe ausgewählt ist.

2. Verfahren nach Anspruch 1, worin der Zeolith wenigstens ein Edelmetall der Platinfamilie in einer Menge, die zwischen 0,01 und 2 Gew.% liegt, und wenigstens ein zusätzliches Metall, das aus der durch Zinn, Germanium, Blei und Indium bestehenden Gruppe ausgewählt ist, in einer Menge zwischen 0,005 und 0,60 Gew.% für Zinn und in einer Menge, zwischen 0,005 und 0,70 Gew.% für Germanium, Blei und Indium, enthält.

3. Verfahren nach einem der Ansprüche 1 und 2, worin der Zeolith wengistens ein Edelmetall der Platinfamilie in einer Menge, die zwischen 0,1 und 0,5 Gew.% liegt und wenigstens ein zusätzliches Metall, das aus der durch Zinn, Germanium, Blei und Indium bestehenden Gruppe ausgewählt ist, in einer Menge zwischen 0,01 und 0,5 Gew.% für Zinn und in einer Menge zwischen 0,01 und 0,60 Gew.% für Germanium, Blei oder Indium enthält.

4. Verfahren nach einem der Ansprüche 1 bis 3, worin der Zeolith als zusätzliche Metalle Zinn und wenigstens ein Metall enthält, das aus der durch Germanium, Blei und Indium bestehenden Gruppe ausgewählt ist, wobei der Gesamtgehalt an zusätzlichen Metallen des Zeolithes zwischen 0,02 und 1,20 Gew.% liegt.

5. Verfahren nach einem der Ansprüche 1 bis 4, das außerdem eine Matrix umfaßt.

6. Verfahren nach Anspruch 5, umfassend 1 bis 99 Gew.% Zeolith und 99 bis 1 % Matrix.

7. Verfahren nach einem der Ansprüche 5 und 6, worin die Matrix Aluminiumoxid ist.
